# FASCICULE DE BREVET EUROPEEN

(11) **EP 0 745 378 B1**
(45) Date de publication et mention de la délivrance du brevet: **11.06.1997**
(21) Numéro de dépôt: 96401077.1
(22) Date de dépôt: 15.05.1996
(51) Int. Cl.: A61K 7/48, A61K 7/06, A61K 7/00, A61K 7/08, A61K 7/02

(54) **Composition solide à base d'amidon contenant des particules creuses thermoplastiques expansées et son utilisation en application topique**
Festes Produkt auf Basis von Stärke, das thermoplastische expandierte hohle Teilchen enthält sowie seiner Anwendung zur topischen Verwendung
Starch solid composition containing thermoplastic expanded hollow particles and its use in topical application

(30) Priorité: 29.05.1995 FR 9506321
(43) Date de publication de la demande: 04.12.1996
(73) Titulaire: L'OREAL, 75008 Paris (FR)
(72) Inventeur: Roulier, Véronique, 75002 Paris (FR); Mellul, Myriam, 94240 l'Hay-Les-Roses (FR); Gabin, Gérard, 75008 Paris (FR); Holz, Katrin, 1012 Lausanne (CH)
(74) Mandataire: Miszputen, Laurent

(56) Documents cités:
- EP-A- 0 486 394
- EP-A- 0 544 349
- EP-A- 0 605 284
- WO-A-92/08759
- FR-A- 1 594 256

## Description

La présente invention concerne des compositions solides expansées et extrudées dont la matrice est constituée d'un réseau alvéolaire amidonné et contient des particules creuses thermoplastiques expansées ainsi que leurs utilisations en application topique.

On connaît dans l'industrie alimentaire des produits expansés à base d'amidon et d'ingrédients comestibles, obtenus par extrusion dans un ou plusieurs extrudeurs mono- ou bi-vis notamment les snacks apéritifs, les chips, les corn-flakes, les produits pour petit-déjeuner à base de céréales, les biscuits.

La demanderesse a découvert de manière surprenante de nouvelles formes galéniques à usage cosmétique ou dermatologique sous la forme d'une composition solide expansée et extrudée dont la matrice est constituée d'un réseau alvéolaire amidonné, susceptible d'être obtenue par mélange, malaxage, expansion et extrusion dans un extrudeur bi-vis, d'au moins (a) un produit amidonné ; (b) de particules creuses thermoplastiques expansées de polymère ou copolymère formé à partir d'un monomère ou d'un mélange de monomères à insaturation éthylènique et (c) d'eau.

La demanderesse a découvert par ailleurs que les compositions de l'invention pouvaient être obtenues par extrusion/expansion, de façon inattendue, à des températures inférieures à 100°C et contenir ainsi des substances cosmétiques ou dermatologiques thermosensibles ou instables à des températures supérieures à 100°C.

Les compositions selon l'invention peuvent constituer en elles-mêmes de nouvelles formes de produits de maquillage tels que des poudres visages, des fards à paupières ou des fards à joues, de nouvelles formes de produits pour l'hygiène tels que des shampooings secs ou pour le soin tels que des produits de démaquillage. Elles présentent l'aspect de boudins, de pellets, de feuilles ou de flocons expansés, pouvant contenir suffisamment de charges pour obtenir un bon délitage et des qualités de douceur satisfaisantes.

Elles peuvent en outre contenir des quantités importantes de phase grasse permettant d'améliorer le confort et de faciliter leur application sur la peau, par exemple, en pouvant les appliquer directement sans l'intermédiaire d'un outil de maquillage (pinceau, éponge, houppette).

La demanderesse a découvert de façon inattendue que l'introduction de particules creuses thermoplastiques expansées de polymère ou copolymère d'un monomère ou d'un mélange de monomères à insaturation éthylènique, dans une matrice constituée d'un réseau alvéolaire amidonné ne diminuait pas le taux d'expansion et permettait l'introduction de fortes quantités de corps gras tels que des huiles et/ou des cires.

Les compositions selon l'invention peuvent notamment contenir, dans des quantités importantes des cires conférant des propriétés de tenue de film, de glissant et de mat. Les poudres compactées habituellement utilisées pour le maquillage ne peuvent pas contenir des quantités importantes de corps gras tels que des cires (plus de 10% en poids). Leur incorporation dans des poudres conduit à des produits qui cirent et qui ne peuvent être délités.

Les particules creuses expansées de polymère ou de copolymère formé à partir d'un monomère ou mélange de monomères à insaturation éthylènique sont des charges de très faible densité inférieure à 0,1 g.cm⁻³ et connues pour leur qualités de douceur et d'absorption des corps gras dans les compositions de maquillage ou de soin. Selon la demande de brevet EP-A-0 486 394, ces particules ont été utilisées pour la préparation de poudres de maquillage coulées dans des coupelles. Elles ont été également utilisées selon la demande EP-A-0 605 284 dans des compositions de maquillage du type coulé ou compacté contenant une forte concentration de charges pulvérulentes de densité légère et une phase grasse ; lesdites compositions étant préparées dans un mélangeur extrudeur-cuiseur à une ou deux vis. Mais ces particules sont connues également par le fait qu'elles sont difficilement compactables.

Par charge difficilement compactable, on entend une matière première qui à partir d'un certain pourcentage qui dépendra de la matière en question, ne peut être compactée au moyen d'une presse mécanique. Ces types de charge ne peuvent pas être utilisées en général dans les produits de maquillage sous forme de poudre compactée. Les produits contenant ce type de charges en faible quantités, ne présentent pas une bonne intégrité au stockage, une bonne solidité aux chocs et/ou une surface plane convenable.

Les compositions selon l'invention, par leur nouvelle structure alvéolaire amidonnée, peuvent donc contenir ces charges difficilement compactables conférant un toucher très doux et non-gras sans présenter les inconvénients des compositions de maquillage de l'art antérieur contenant ce types de charges.

Les compositions de l'invention par leur nouvelle structure alvéolaire amidonnée peuvent constituer de nouvelles formes de produits de maquillage dont les qualités cosmétiques sont originales notamment pour leur douceur et leur légéreté dues à l'incorporation de charges de faible densité.

Les compositions selon l'invention peuvent se présenter sous forme de boudins, pellets, de feuilles ou flocons expansés appliqués directement sur la peau ou le visage ou être réduites en poudre et utilisées telles quelles, de façon classique, comme poudre de maquillage.

Elles peuvent se présenter sous forme de poudre de soin et/ou d'hygiène, appliquée directement sur la peau, le cuir chevelu ou les cheveux par exemple des shampooings à sec ou des poudres libres pour le soin du corps.

Elles peuvent également se présenter sous forme de boudins, de pellets, de feuilles ou de flocons expansés, stockés à l'état sec, et être très facilement réhydratables après immersion dans un milieu aqueux pour reconstituer des formulations pour le maquillage telles que des fonds de teint ou des formulations pour le soin ou l'hygiène telles que des crèmes, des laits, des bains moussants, des gels, des shampooings. Il est ainsi possible d'incorporer dans ces formes galéniques, stables au stockage à des températures inférieures à 45°C, des actifs cosmétiques hydrosensibles.

Les compositions selon l'invention,stockées à sec et destinées à être réhydratées au moment de l'emploi pour reconstituer les formulations cosmétiques telles que citées ci-dessus, présentent l'avantage par rapport aux formes galléniques réhydratables classiques, d'être très facilement réhydratable et dans le cadre des compositions de nettoyage, notamment les shampooings, d'être moins agressive du fait que les tensio-actifs sont intégrés dans une matrice expansée amidonnée.

Les compositions selon l'invention sont susceptibles d'être obtenues par mélange, malaxage, expansion et extrusion dans un extrudeur bi-vis, d'au moins : (a) un produit amidonné choisi parmi les farines de céréale ou de pomme de terre ; les amidons purs ; les amidons modifiés au niveau du rapport amylose/amylopectine ; les amidons réticulés et les amidons modifiés par un groupe fonctionnel ; (b) de particules creuses thermoplastiques expansées de polymère ou copolymère formé à partir d'un monomère ou d'un mélange de monomères à insaturation éthylènique ; et (c) d'eau.

Les particules utilisables selon l'invention peuvent être réalisées à partir de monomères à insaturation éthylènique, non-toxiques et non-irritants pour la peau.

Les particules de l'invention peuvent être obtenues, par exemple, selon les procédés des brevets et demandes de brevet EP-56219, EP-348372, EP-486080, EP-320473, EP-112807 et US-3615972.

La cavité interne des particules contient en principe un gaz qui peut être de l'air, de l'azote ou un hydrocarbure comme l'isobutane ou l'isopentane.

Parmi les monomères utilisés pour la réalisation des particules creuses thermoplastiques expansées de l'invention, on peut citer les esters d'acide méthacrylique ou acrylique tels que l'acrylate ou le méthacrylate de méthyle ; le chlorure de vinylidène ; l'acrylonitrile ; le styrène et ses dérivés ; le butadiène et ses dérivés ; leurs mélanges.

On peut par exemple utiliser les polymères ou copolymères d'acrylate ou méthacrylate de méthyle, les copolymères formés à partir de styrène et d'acrylonitrile, les copolymères de chlorure de vinylidène et d'acrylonitrile ou de chlorure de vinyle.

On utilise de préférence un polymère ou copolymère contenant : de 0 % à 60 % de chlorure de vinylidène, de 20% à 90 % d'acrylonitrile et de 0 % à 50 % d'un monomère (méth)acrylique ou styrènique, la somme des pourcentages (en poids) étant égale à 100. Le monomère (méth)acrylique est par exemple le (méth)acrylate de méthyle ou d'éthyle. Le monomère styrènique est par exemple le styrène ou le α-méthyl-styrène.

Plus préférentiellement, les particules utilisées dans la présente invention sont des particules creuses d'un copolymère expansé de chlorure de vinylidène et d'acrylonitrile ou de chlorure de vinylidène, d'acrylonitrile et de méthacrylate de méthyle. Ces particules peuvent être sèches ou hydratées.

De façon avantageuse, les particules de l'invention ont une granulométrie allant de 1 µm à 100 µm et encore mieux allant de 5 µm à 60 µm, et encore mieux de 10 µm à 50 µm.

De façon préférentielle, la masse volumique des particules est choisie dans la gamme allant de 15 kg/m³ à 200 kg/m³ et mieux de 40 kg/m³ à 120 kg/m³, et encore mieux de 60 kg/m³ à 80 kg/m³.

Les particules utilisables dans l'invention sont par exemple les microsphères de terpolymère expansé de chlorure de vinylidène, d'acrylonitrile et de méthacrylate, vendues sous la marque EXPANCEL par la société Nobel Casco sous les références 551 DE 50 (granulométrie d'environ 40 µm), 551 DE 20 (granulométrie d'environ 30µm et masse volumique d'environ 65 kg/m³), 551 DE 12 (granulométrie d'environ 12 µm), 551 DE 80 (granulométrie d'environ 80 µm), 461 DE 50 (granulométrie d'environ 50 µm). On peut aussi utiliser des microsphères formées du même terpolymère expansé ayant une granulométrie d'environ 18 µm et une masse volumique d'environ 70 kg/m³, appelées ci-dessous EL 23.

Les particules creuses thermoplastiques expansées sont présentes dans les compositions de l'invention dans des concentrations allant, préférentiellement, de 2 à 30% en poids par rapport au poids total de la composition.

Les produits riches en amidon utilisés dans les compositions selon l'invention sont choisis de préférence parmi les farines de céréale telles que les farines de blé, de maïs, de riz, d'avoine, de germes de blé ou la farine de pomme de terre ; les amidons purs couramment utilisés en alimentaire tels que les amidons de maïs, de pomme de terre, de tapioca, d'avoine ; les amidons modifiés au niveau du rapport amylose/amylopectine tels que le produit HYLON VII vendu par la société AMYLUM ; les amidons modifiés par réticulation ou par un groupe fonctionnel tels que l'amidon de maïs réticulé vendu sous le nom RESISTAMYL E2 par la société AMYLUM, l'amidon de maïs faiblement quaternisé vendu sous le nom MYPLUS W7 par la société AMYLUM, l'amidon de pomme de terre vendu sous le nom SUPRAMYL P 60 par AMYLUM ou l'amidon de maïs hydroxypropylé vendu sous le nom MERIGEL EF6 par AMYLUM.

La matrice constituée du réseau alvéolaire amidonné formé à partir de ces produits riches en amidon est présente dans les compositions selon l'invention, de préférence, dans une proportion allant de 70 à 98% en poids par rapport au poids de la composition.

Les compositions selon l'invention présente une teneur en eau de préférence inférieure ou égale à 5% en poids et plus particulièrement allant de 1 à 2 % en poids par rapport au poids de la composition.

Les compositions selon l'invention peuvent contenir en plus une phase grasse. Cette phase grasse peut comprendre des huiles et/ou des cires d'origine animale, végétale, minérale ou synthétique, seules ou en mélanges.

Parmi les huiles utilisables, on peut citer l'huile de vison, l'huile de tortue, l'huile de soja, l'huile de pépins de raison, l'huile de sésame, l'huile de maïs, l'huile de colza, l'huile de tournesol, l'huile de coton, l'huile d'avocat, l'huile d'olive, l'huile de ricin, l'huile de jojoba, l'huile d'arachide; les huiles d'hydrocarbures, telles que les huiles de paraffine, le squalane, la vaseline; les esters gras, tels que le myristate d'isopropyle, le palmitate d'isopropyle, le stéarate de butyle, le laurate d'hexyle, l'isononate d'isononyle, le palmitate de 2-éthyl-hexyle, le laurate de 2-hexyl-décyle, le palmitate de 2-octyl-décyle, le myristate ou le lactate de 2-octyldodécyle, le succinate de 2-éthyl-hexyle, le malate de diisostéaryle, le triisostéarate de glycérine ou de diglycérine; les huiles de silicone telles que les polyméthylsiloxanes, les polyméthylphénylsiloxanes, les polysiloxanes modifiés par des acides gras, des alcools gras ou des polyoxyalkylènes, les silicones fluorées, les huiles perfluorées; les acides gras supérieurs tels que l'acide myristique, l'acide palmitique, l'acide stéarique, l'acide béhénique, l'acide oléylique, l'acide linoléique, l'acide linolénique ou l'acide isostéarique; les alcools gras supérieurs tels que le cétanol, l'alcool stéarylique ou l'alcool oléique.

Parmi les cires utilisables, on peut citer les cires d'abeille, les cires de lanoline et les cires d'insectes de Chine; les cires de Carnauba, de Candelilla, d'ouricurry, les cires de fibres de liège, les cires de canne à sucre, les cires du Japon, les cires de jojoba hydrogénées et les huiles hydrogénées telles que l'huile de tournesol hydrogénée, l'huile de ricin hydrogénée, l'huile de coprah hydrogénée et la lanoline hydrogénée; les paraffines, les cires microcristallines, les cires de Montan et les ozokérites; les cires de polyéthylène, les cires obtenues par la synthèse de Fischer-Tropsch, les copolymères cireux ainsi que leurs esters, et les cires de silicone telles que les polyalcoxy et polyalkylsiloxanes.

La phase grasse est présente dans des proportions allant, préférentiellement, de 2 à 30% en poids et plus particulièrement de 5 à 15% en poids par rapport au poids total de la composition.

La phase grasse peut, en outre, comprendre des additifs tels que des actifs cosmétiques lipophiles et/ou des ingrédients liposolubles généralement utilisés en cosmétique comme les parfums. De préférence, ces additifs peuvent être présents en une quantité de 0-20% par rapport au poids total de la phase grasse.

Les compositions selon l'invention peuvent contenir en plus des pigments, de préférence en une quantité de 0-50% par rapport au poids total de la composition finale. Ces pigments peuvent être choisis parmi les pigments minéraux, les pigments organiques et les pigments nacrés.

Parmi les pigments minéraux, on peut citer, par exemple le dioxyde de titane (rutile ou anatase), éventuellement traité en surface; les oxydes de fer noir, jaune, rouge et brun; le violet de manganèse; le bleu outremer; l'oxyde de chrome éventuellement hydraté ; le bleu ferrique.

Parmi les pigments organiques, on peut citer, par exemple, les pigments D & C red, D & C orange, D & C yellow, le noir de carbone, et les laques à base de carmin de cochenille.

Les pigments nacrés peuvent être choisis, notamment, parmi les pigments nacrés blancs tels que le mica recouvert d'oxyde de titane ou l'oxychlorure de bismuth; les pigments nacrés colorés tels que le micatitane avec des oxydes de fer, le micatitane avec du bleu ferrique ou de l'oxyde de chrome, le micatitane avec un pigment organique du type précité, ainsi que les pigments à base d'oxychlorure de bismuth.

Les compositions selon l'invention peuvent contenir également d'autres charges minérales ou organiques habituellement utilisés dans les produits de maquillage telles que, par exemple, le talc, les micas, le kaolin, la silice, les oxydes de zinc et de titane, le carbonate de calcium, le carbonate et l'hydrocarbonate de magnésium, les poudres de polymères synthétiques non expansées, les savons métalliques dérivés d'acide carboxylique en C₈-C₂₂. Elles sont présentes dans des concentrations allant préférentiellement de 0 à 50% en poids par rapport au poids de la composition.

Les compositions selon l'invention peuvent également contenir un ou plusieurs tensio-actifs non ioniques, anioniques, cationiques ou amphotères, habituellement utilisés en cosmétique. La quantité d'agent tensioactif utilisée est de préférence de 2 à 30% par rapport au poids total de la composition.

Les compositions selon l'invention selon l'invention peuvent également contenir en plus des actifs cosmétiques hydrosolubles.

Parmi les actifs cosmétiques, on peut citer les agents antioxydants ou anti-radicaux libres; les agents hydratants ou humectants tels que la glycérine et le collagène; les agents filtres UV tels que la benzophénone. Ces actifs hydrosolubles peuvent être présents dans la composition finale en une quantité de 0 à 20%, de préférence 5 à 15%. en poids. Parmi les actifs thermosensibles, on peut citer les vitamines telles que la vitamine E.

Bien entendu, l'homme de l'art veillera à choisir les éventuels composés complémentaires et/ou leurs quantités de manière telle que les propriétés avantageuses attachés intrinsèquement à la composition conforme à l'invention ne soient pas, ou substantiellement pas, altérées par la ou les adjonctions envisagées.

La présente invention concerne également un procédé de préparation d'une composition telle que définie précédemment, caractérisé par le fait que celle-ci est obtenue à partir du produit riche en amidon, des particules creuses thermoplastiques et des constituants additionnels eventuels tels qu'indiqués ci-dessus en présence d'eau, par mélange, malaxage et expansion dans un extrudeur bi-vis.

L'extrudeur utilisé pour le procédé de l'invention est choisi parmi les extrudeurs bi-vis tel que celui décrit dans la demande FR94-00756.

Les matières premières sont introduites, à l'entrée de l'extrudeur bi-vis, dans la zone d'alimentation à température ambiante, de préférence à environ 20°C, , puis sont amenées dans la zone de transport à une température ,de préférence, à environ 50°C, puis sont malaxées et comprimées dans diverses zones de l'extrudeur maintenues à une température inférieure à 100°C allant, préférentiellement, de 60 à 80°C; la masse obtenue est transportée vers la sortie de l'extrudeur et extrudée au travers d'une filière pour y subir une expansion.; les produits extrudés sont ensuite, si nécessaire, séchés selon un procédé classique de séchage (four, étuve ventilée).

Pendant la phase de mélange, le produit riche en amidon se gélatinise et forme, après extrusion, un réseau alvéolaire constituant la matrice des produits expansés finaux.

Un avantage surprenant du procédé de l'invention est que l'utilisation des particules creuses thermoplatiques de polymère ou copolymère de monomère ou de mélange de monomères à insaturation éthylènique, permet d'obtenir une expansion des compositions à des températures de l'ordre de 60-80°C. Ce procédé permet d'obtenir des produits expansés pouvant contenir des substances cosmétiques ou dermatologiques thermosensibles à des températures supérieures à 100°C telles que des vitamines ou certaines huiles se dégradant à plus de 100°C.

Un autre objet de l'invention consiste en de nouvelles compositions cosmétiques ou dermatologiques, caractérisées par le fait qu'elles sont constituées par une composition solide expansée telle que définie ci-dessus.

Ces compositions peuvent se présenter sous la forme de boudins, pellets, feuilles ou flocons expansés ou bien être réduites à l'état de poudre.

Ces compositions peuvent être des produits pour le maquillage. Elles peuvent être appliquées sur le visage soit directement soit par l'intermédiaire d'un outil de maquillage tel qu'un pinceau, une houpette ou un tampon applicateur. Elles peuvent être stockées à l'état sec et , au moment de l'utilisation, réhydratées après immersion dans l'eau pour reconstituer une formulation aqueuse de maquillage liquide ou semi-liquide telle qu'un fonds de teint.

Les compositions selon l'invention peuvent être des produits pour le soin et/ou l'hygiène de la peau, des muqueuses, du cuir chevelu ou des cheveux.

Elles peuvent se présenter sous forme de poudre et appliquées directement sur la peau, le cuir chevelu ou les cheveux comme par exemple un shampooing sec ou une poudre libre pour le soin du corps.Ces compositions peuvent également se présenter sous forme de poudre ou bien de boudins, pellets ou flocons expansés, réhydratables après immersion dans l'eau de manière à reconstituer une formulation aqueuse pour le soin et/ou l'hygiène telle qu'une crème, un lait, un gel, un bain moussant, un shampooing.

Les exemples qui suivent servent à illustrer l'invention sans pour autant en limiter la portée.

### EXEMPLES

### Exemple 1 Shampooing sec sous forme de poudre à réhydrater

Le produit final a pour formulation suivante :

| | |
|---|---|
| - Farine de blé | 35,0% en poids |
| - Amidon de maïs | 35,0% en poids |
| - Particules expansées de copolymère de chlorure de vinylidène/acrylonitrile/méthacrylate de méthyle vendu sous le nom EXPANCEL 550DE par la Société CASCO NOBEL | 15,0% en poids |
| - Lauryl-éther sulfate de sodium | 15,0% en poids |

### MODE OPERATOIRE :

Les matières premières sont introduites à l'entrée de l'extrudeur à une température de 30°C. Elles sont ensuite amenées dans la zone de transport à une température ,de préférence, à environ 50°C, puis sont malaxées et comprimées dans diverses zones de l'extrudeur maintenues à 60-80°C. La masse ainsi malaxée et comprimée, est transportée vers la sortie de l'extrudeur et extrudée au travers d'une filière de 5 mm de diamètre. La vitesse de rotation des vis est de 500 tours/minute. Les boudins obtenus à la sortie de la filière sont réduits à l'état de poudre au moyen d'un broyeur à broches classique placé à la sortie de l'extrudeur.

### Exemple 2 Masque pour le visage sous forme de « feuilles expansées » à réhydrater

Le produit final a pour formulation suivante :

| | |
|---|---|
| - Farine de blé | 24,0% en poids |
| - Amidon | 24,0% en poids |
| - Particules expansées de copolymère de chlorure de vinylidène/acrylonitrile/méthacrylate de méthyle vendu sous le nom EXPANCEL 550DE par la Société CASCO NOBEL | 10,0% en poids |
| - Silice vendue sous le nom SB700 par la Société MAPRECOS | 40,0% en poids |
| - Vitamine E | 2,0% en poids |

### MODE OPERATOIRE :

Les matières premières sont introduites à l'entrée d'un extrudeur bi-vis à une température de 30°C. Elles sont ensuite amenées dans la zone de transport à une température ,de préférence, à environ 50°C, puis sont malaxées et comprimées dans diverses zones de l'extrudeur maintenues à 60-80°C. La masse ainsi malaxée et comprimée, est transportée vers la sortie de l'extrudeur et extrudée au travers d'une filière de 5 mm de diamètre. Les bandes expansées, obtenues à la sortie de la filière, sont coupées sous forme de feuilles à la dimension souhaitée.

## Revendications

1. Composition solide expansée et extrudée dont la matrice est constituée d'un réseau alvéolaire amidonné, susceptible d'ëtre obtenue par mélange, malaxage, expansion et extrusion dans un extrudeur bi-vis, d'au moins : (a) un produit amidonné choisi parmi les farines de céréale ou de pomme de terre ; les amidons purs ; les amidons modifiés au niveau du rapport amylose/amylopectine ; les amidons réticulés et les amidons modifiés par un groupe fonctionnel ; (b) des particules creuses thermoplastiques expansées de polymère ou copolymère formé à partir d'un monomère ou d'un mélange de monomères à insaturation éthylènique ; et (c) de l'eau.

2. Composition selon la revendication 1, selon laquelle le produit amidonné, les particules creuses thermoplastiques expansées et l'eau sont introduits à l'entrée de l'extrudeur bi-vis, dans la zone d'alimentation à température ambiante, puis puis sont malaxées et comprimées dans diverses zones de l'extrudeur maintenues à une température inférieure à 100°C ; la masse obtenue est transportée vers la sortie de l'extrudeur et extrudée au travers d'une filière pour y subir une expansion ; les produits extrudés sont ensuite éventuellement séchés selon un procédé classique de séchage.

3. Composition selon la revendication 1 ou 2, caractérisée par le fait qu'elle se présente sous forme de boudin expansé, pellet expansé, de feuille expansée ou de flocon expansé.

4. Composition selon l'une quelconque des revendications 1 à 3; caractérisée par le fait que les particules creuses thermoplastiques expansées sont des particules de polymère ou de copolymère expansé formé à partir d'un monomère ou d'un mélange de monomères choisis dans le groupe formé par les esters d'acide méthacrylique ou acrylique ; le chlorure de vinylidène ; l'acrylonitrile ; le styrène et ses dérivés ; le butadiène et ses dérivés.

5. Composition selon l'une quelconque des revendications 1 à 4, caractérisée par le fait que les particules creuses thermoplastiques sont des particules de polymère ou copolymère d'acrylate ou méthacrylate de méthyle, de copolymère de styrène et d'acrylonitrile ou bien de copolymère de chlorure de vinylidène et d'acrylonitrile ou de chlorure de vinyle.

6. Composition selon l'une quelconque des revendications 1 à 5, caractérisée par le fait que le polymère ou copolymère contient de 0 % à 60 % de motifs dérivés du chlorure de vinylidène, de 20 % à 90 % d'acrylonitrile et de 0 % à 50 % de monomère acrylique ou styrénique, la somme des pourcentages (en poids) étant égale à 100.

7. Composition selon la revendication 6, caractérisée par le fait que les particules creuses thermoplastiques expansées sont des particules creuses d'un copolymère expansé de chlorure de vinylidène et d'acrylonitrile ou un terpolymère expansé de chlorure de vinylidène, d'acrylonitrile et de méthacrylate de méthyle.

8. Composition selon l'une quelconque des revendications 1 à 7, caractérisée par le fait que les particules ont une granulométrie de 1 à 100 µm.

9. Composition selon l'une quelconque des revendications 1 à 8, caractérisée par le fait que les particules creuses thermoplastiques ont une masse volumique allant de 15 à 200 kg/cm³.

10. Composition selon l'une quelconque des revendications 1 à 9, caractérisée par le fait qu'elle contient de 2 à 30% en poids de charge par rapport au poids total de la composition.

11. Composition selon l'une quelconque des revendications 1 à 10, dans laquelle la matrice constituée d'un réseau alvéolaire amidonné représente de 70 à 98% en poids du poids total de la composition.

12. Composition selon l'une quelconque des revendications 1 à 11, dans laquelle la teneur en eau est inférieure à 5% en poids par rapport au poids total de la composition.

13. Composition selon la revendication 12, dans laquelle la teneur en eau varie de 1 à 2% en poids par rapport au poids total de la composition.

14. Composition selon l'une quelconque des revendications 1 à 13, caractérisée par le fait qu'elle contient en plus une phase grasse.

15. Composition selon la revendication 14, comprenant 2 à 30% en poids de phase grasse.

16. Composition selon l'une quelconque des revendication 1 à 15, caractérisée par le fait qu'elle contient des adjuvants choisis parmi les pigments, les charges minérales ou organiques, les tensio-actifs, les actifs liposolubles, les additifs lipososolubles habituellement utilisés en cosmétique, les anti-oxydants, les anti-radicaux libres, les hydratants, les humectants, les filtres solaires.

17. Composition selon l'une quelconque des revendication 1 à 15, caractérisée par le fait qu'elle est réduite à l'état de poudre.

18. Composition à usage cosmétique ou dermatologique, caractérisée par le fait qu'elle est constituée par une composition solide expansée et extrudée telle que définie selon l'une quelconque des revendications 1 à 17.

19. Composition selon la revendication 18, caractérisée par le fait qu'elle est un produit pour le maquillage.

20. Composition selon la revendication 18, caractérisée par le fait qu'elle est un produit pour le soin et/ou l'hygiène de la peau, des muqueuses, du cuir chevelu ou des cheveux.

21. Procédé de préparation d'une composition solide expansée et extrudée dont la matrice est constituée d'un réseau alvéolaire amidonné, caractérisé par le fait que ladite composition est préparée par mélange, malaxage, expansion et extrusion dans un extrudeur bi-vis, d'au moins : (a) un produit amidonné choisi parmi les farines de céréale ou de pomme de terre ; les amidons purs ; les amidons modifiés au niveau du rapport amylose/amylopectine ;les amidons réticulés et les amidons modifiés par un groupe fonctionnel ; (b) de particules creuses thermoplastiques expansées de polymère ou copolymère formé à partir d'un monomère ou d'un mélange de monomères à insaturation éthylènique ; et (c) d'eau.

22. Procédé selon la revendication 20, caractérisé par le fait que les matières premières sont introduites à l'entrée de l'extrudeur bi-vis, dans la zone d'alimentation à température ambiante, puis puis sont malaxées et comprimées dans diverses zones de l'extrudeur maintenues à une température inférieure à 100°C ; la masse obtenue est transportée vers la sortie de l'extrudeur et extrudée au travers d'une filière pour y subir une expansion ; les produits extrudés sont ensuite éventuellement séchés selon un procédé classique de séchage.

23. Procédé selon la revendication 22, caractérisé par le fait que les zones de malaxage et de compression sont chauffées à une température allant de 60° à 80°C.

## Claims

1. Expanded and extruded solid composition whose matrix consists of a starch-based cellular network, capable of being obtained by the mixing, kneading, expansion and extrusion in a twin-screw extruder of at least: (a) a starch-based product chosen from cereal or potato flours; pure starches; starches modified in respect of the amylose/amylopectin ratio; crosslinked starches and starches modified with a functional group; (b) expanded thermoplastic hollow particles of polymer or copolymer formed from an ethylenically unsaturated monomer or mixture of such monomers; and (c) water.

2. Composition according to Claim 1, according to which the starch-based product, the expanded thermoplastic hollow particles and water are introduced at the entry of the twin-screw extruder into the feeding zone at room temperature, and are then kneaded and compressed in various zones of the extruder which are maintained at a temperature below 100°C; the mass obtained is transported to the exit of the extruder and extruded through a die to undergo an expansion thereat; the extruded products are thereafter, if appropriate, dried according to a standard drying process.

3. Composition according to Claim 1 or 2, characterized in that it takes the form of an expanded cylinder, expanded pellet, expanded leaf or expanded flake.

4. Composition according to any one of Claims 1 to 3, characterized in that the expanded thermoplastic hollow particles are particles of expanded polymer or copolymer formed from a monomer or mixture of monomers chosen from the group composed of methacrylic or acrylic acid esters; vinylidene chloride; acrylonitrile; styrene and its derivatives; and butadiene and its derivatives.

5. Composition according to any one of Claims 1 to 4, characterized in that the thermoplastic hollow particles are particles of methyl acrylate or methacrylate polymer or copolymer, of copolymer of styrene and acrylonitrile or alternatively of copolymer of vinylidene chloride and acrylonitrile or vinyl chloride.

6. Composition according to any one of Claims 1 to 5, characterized in that the polymer or copolymer contains from 0 % to 60 % of units derived from vinylidene chloride, from 20 % to 90 % of acrylonitrile and from 0 % to 50 % of acrylic or styrene monomer, the sum of the percentages (by weight) being equal to 100.

7. Composition according to Claim 6, characterized in that the expanded thermoplastic hollow particles are hollow particles of an expanded copolymer of vinylidene chloride and acrylonitrile, or an expanded terpolymer of vinylidene chloride, acrylonitrile and methyl methacrylate.

8. Composition according to any one of Claims 1 to 7, characterized in that the particles have a particle size of 1 to 100 µm.

9. Composition according to any one of Claims 1 to 8, characterized in that the thermoplastic hollow particles have a density ranging from 15 to 200 kg/cm³.

10. Composition according to any one of Claims 1 to 9, characterized in that it contains from 2 to 30 % by weight of filler relative to the total weight of the composition.

11. Composition according to any one of Claims 1 to 10, in which the matrix consisting of a starch-based cellular network represents from 70 to 98 % by weight of the total weight of the composition.

12. Composition according to any one of Claims 1 to 11, in which the water content is less than 5 % by weight relative to the total weight of the composition.

13. Composition according to Claim 12, in which the water content varies from 1 to 2 % by weight relative to the total weight of the composition.

14. Composition according to any one of Claims 1 to 13, characterized in that it contains, in addition, a fatty phase.

15. Composition according to Claim 14, comprising 2 to 30 % by weight of fatty phase.

16. Composition according to any one of Claims 1 to 15, characterized in that it contains adjuvants chosen from pigments, inorganic or organic fillers, surfactants, fat-soluble active agents, fat-soluble additives customarily used in cosmetics, antioxidants, free-radical scavengers, hydrating agents, humectants and sunscreen agents.

17. Composition according to any one of Claims 1 to 15, characterized in that it is reduced to the powder state.

18. Composition for cosmetic or dermatological use, characterized in that it consists of an expanded and extruded solid composition as defined according to any one of Claims 1 to 17.

19. Composition according to Claim 18, characterized in that it is a make-up product.

20. Composition according to Claim 18, characterized in that it is a product for the care and/or hygiene of the skin, mucosae, scalp or hair.

21. Process for preparing an expanded and extruded solid composition whose matrix consists of a starch-based cellular network, characterized in that the said composition is prepared by the mixing, kneading, expansion and extrusion in a twin-screw extruder of at least: (a) a starch-based product chosen from cereal or potato flours; pure starches; starches modified in respect of the amylose/amylopectin ratio; crosslinked starches and starches modified with a functional group; (b) expanded thermoplastic hollow particles of polymer or copolymer formed from an ethylenically unsaturated monomer or mixture of such monomers; and (c) water.

22. Process according to Claim 20, characterized in that the starting materials are introduced at the entry of the twin-screw extruder into the feeding zone at room temperature, and are then kneaded and compressed in various zones of the extruder which are maintained at a temperature below 100°C; the mass obtained is transported to the exit of the extruder and extruded through a die to undergo an expansion thereat; the extruded products are thereafter, if appropriate, dried according to a standard drying process.

23. Process according to Claim 22, characterized in that kneading and compression zones are heated to a temperature ranging from 60° to 80°C.

## Patentansprüche

1. Feste expandierte und extrudierte Zusammensetzung, deren Matrix aus einem zelligen Netzwerk auf Stärkebasis besteht, die durch Mischen, Verkneten, Expandieren und Extrudieren mindestens folgender Komponenten in einem Zweischneckenextruder erhältlich ist:
(a) eines Stärkematerials, das unter Getreidemehlen und Kartoffelmehl, reinen Stärken, hinsichtlich des Verhältnisses Amylose/Amylopektin modifizierten Stärken, vernetzten Stärken und mit einer funktionellen Gruppe modifizierten Stärken ausgewählt ist,
(b) expandierter hohler thermoplastischer Teilchen aus einem Polymer oder Copolymer, das aus einem ethylenisch ungesättigten Monomer oder einem Gemisch ethylenisch ungesättigter Monomerer hergestellt ist,
und
(c) Wasser.

2. Zusammensetzung nach Anspruch 1, erhältlich durch Einführen des Stärkematerials, der expandierten hohlen thermoplastischen Teilchen und des Wassers in den Einlaß des Zweischneckenextruders in der Zuführungszone bei Raumtemperatur, anschließendes Verkneten und Verpressen in verschiedenen auf einer Temperatur unter 100 °C gehaltenen Zonen des Extruders, Transportieren der erhaltenen Masse zum Ausgang des Extruders und Extrudieren durch eine Düse, wo eine Expandierung erfolgt, sowie ggf. anschließendes Trocknen der extrudierten Produkte nach einem herkömmlichen Trocknungsverfahren.

3. Zusammensetzung nach Anspruch 1 oder 2, dadurch gekennzeichnet, daß sie in Form expandierter Stränge, expandierter Pellets, expandierter Platten oder expandierter Flocken vorliegt.

4. Zusammensetzung nach einem der Ansprüche 1 bis 3, dadurch gekennzeichnet, daß die expandierten hohlen thermoplastischen Teilchen Partikel eines expandierten Polymers oder Copolymers sind, das aus einem Monomer oder einem Gemisch von Monomeren hergestellt ist, die ausgewählt sind unter Estern von Methacrylsäure oder Acrylsäure, Vinylidenchlorid, Acrylnitril, Styrol und seinen Derivaten sowie Butadien und seinen Derivaten.

5. Zusammensetzung nach einem der Ansprüche 1 bis 4, dadurch gekennzeichnet, daß die hohlen thermoplastischen Teilchen Partikel eines Polymers oder Copolymers von Methylacrylat oder Methylmethacrylat, eines Copolymers von Styrol und Acrylnitril oder eines Copolymers von Vinylidenchlorid und Acrylnitril oder Vinylchlorid sind.

6. Zusammensetzung nach einem der Ansprüche 1 bis 5, dadurch gekennzeichnet, daß das Polymer oder Copolymer 0 bis 60 % von Vinylidenchlorid abgeleitete Einheiten, 20 bis 90 % von Acrylnitril abgeleitete Einheiten und 0 bis 50 % von einem Acrylmonomer oder einem Styrolmonomer abgeleitete Einheiten enthält, wobei die Summe der Prozentanteile (gewichtsbezogen) gleich 100 ist.

7. Zusammensetzung nach Anspruch 6, dadurch gekennzeichnet, daß die expandierten hohlen thermoplastischen Teilchen hohle Partikel eines expandierten Copolymers von Vinylidenchlorid und Acrylnitril oder eines expandierten Terpolymers von Vinylidenchlorid, Acrylnitril und Methylmethacrylat sind.

8. Zusammensetzung nach einem der Ansprüche 1 bis 7, dadurch gekennzeichnet, daß die Teilchen eine Teilchengröße von 1 bis 100 µm aufweisen.

9. Zusammensetzung nach einem der Ansprüche 1 bis 8, dadurch gekennzeichnet, daß die hohlen thermoplastischen Teilchen eine Dichte von 15 bis 200 kg/cm³ aufweisen.

10. Zusammensetzung nach einem der Ansprüche 1 bis 9, dadurch gekennzeichnet, daß sie 2 bis 30 Gew.-% Füllstoff, bezogen auf das Gesamtgewicht der Zusammensetzung, enthält.

11. Zusammensetzung nach einem der Ansprüche 1 bis 10, bei der die aus einem zelligen Netzwerk auf Stärkebasis bestehende Matrix 70 bis 98 Gew.-%, bezogen auf das Gesamtgewicht der Zusammensetzung, ausmacht.

12. Zusammensetzung nach einem der Ansprüche 1 bis 11, bei welcher der Wassergehalt, bezogen auf das Gesamtgewicht der Zusammensetzung, weniger als 5 Gew.-% beträgt.

13. Zusammensetzung nach Anspruch 12, bei welcher der Wassergehalt, bezogen auf das Gesamtgewicht der Zusammensetzung, 1 bis 2 Gew.-% beträgt.

14. Zusammensetzung nach einem der Ansprüche 1 bis 13, dadurch gekennzeichnet, daß sie ferner eine Fettphase enthält.

15. Zusammensetzung nach Anspruch 14, die 2 bis 30 Gew.-% Fettphase enthält.

16. Zusammensetzung nach einem der Ansprüche 1 bis 15, dadurch gekennzeichnet, daß sie Hilfsstoffe enthält, die ausgewählt sind unter Pigmenten, anorganischen oder organischen Füllstoffen, grenzflächenaktiven Stoffen, fettlöslichen Wirkstoffen, fettlöslichen Zusätzen, die gewohnlich in der Kosmetik verwendet werden, Antioxidationsmitteln, Mitteln gegen freie Radikale, Hydratationsmitteln, Befeuchtungsmitteln und Sonnenschutzfiltern.

17. Zusammensetzung nach einem der Ansprüche 1 bis 15, dadurch gekennzeichnet, daß sie zu einem Pulver zerkleinert ist.

18. Zusammensetzung zur kosmetischen oder dermatologischen Anwendung, dadurch gekennzeichnet, daß sie aus einer festen expandierten und extrudierten Zusammensetzung nach einem der Ansprüche 1 bis 17 besteht.

19. Zusammensetzung nach Anspruch 18, dadurch gekennzeichnet, daß sie ein Produkt zum Schminken ist.

20. Zusammensetzung nach Anspruch 18, dadurch gekennzeichnet, daß sie ein Produkt zur Pflege und/oder zur Hygiene der Haut, der Schleimhäute, der Kopfhaut oder der Haare ist.

21. Verfahren zur Herstellung einer festen expandierten und extrudierten Zusammensetzung, deren Matrix aus einem zelligen Netzwerk auf Stärkebasis besteht, dadurch gekennzeichnet, daß die Zusammensetzung durch Mischen, Verkneten, Expandieren und Extrudieren in einem Zweischneckenextruder aus mindestens folgenden Komponenten hergestellt wird:
(a) einem Stärkematerial, das unter Getreidemehlen und Kartoffelmehl, reinen Stärken, hinsichtlich des Verhältnisses Amylose/Amylopektin modifizierten Stärken, vernetzten Stärken und mit einer funktionellen Gruppe vernetzten Stärken ausgewählt ist,
(b) expandierten hohlen thermoplastischen Teilchen aus einem Polymer oder Copolymer, das aus einem ethylenisch ungesättigten Monomer oder einem Gemisch ethylenisch ungesättigter Monomerer hergestellt ist,
und
(c) Wasser.

22. Verfahren nach Anspruch 21, dadurch gekennzeichnet, daß die Ausgangsmaterialien in den Einlaß des Zweischneckenextruders in der Zuführungszone bei Raumtemperatur eingeführt werden und anschließend in verschiedenen auf einer Temperatur unter 100 °C gehaltenen Zonen des Extruders verknetet und verpreßt werden und die erhaltene Masse zum Ausgang des Extruders transportiert und durch eine Düse extrudiert wird, wo eine Expandierung erfolgt, wonach die extrudierten Produkte ggf. nach einem herkömmlichen Trocknungsverfahren getrocknet werden.

23. Verfahren nach Anspruch 22, dadurch gekennzeichnet, daß die Zonen, wo das Verkneten und das Verpressen erfolgen, auf eine Temperatur von 60 bis 80 °C erwärmt werden.
